(19) Europäisches Patentamt
European Patent Office
Office européen des brevets

(11) Veröffentlichungsnummer: **0 299 295 B1**

(12) **EUROPÄISCHE PATENTSCHRIFT**

(45) Veröffentlichungstag der Patentschrift: **16.09.92**

(51) Int. Cl.[5]: **C07C 69/33**, B01F 17/34, A61K 7/00, C07C 67/03, C07C 69/675

(21) Anmeldenummer: **88110527.4**

(22) Anmeldetag: **01.07.88**

Die Akte enthält technische Angaben, die nach dem Eingang der Anmeldung eingereicht wurden und die nicht in dieser Patentschrift enthalten sind.

# (54) Polyglycerinester der Wollfettsäure, Verfahren zu ihrer Herstellung und ihre Verwendung als Emulgatoren.

(30) Priorität: **14.07.87 DE 3723237**

(43) Veröffentlichungstag der Anmeldung:
**18.01.89 Patentblatt 89/03**

(45) Bekanntmachung des Hinweises auf die Patenterteilung:
**16.09.92 Patentblatt 92/38**

(84) Benannte Vertragsstaaten:
**BE CH FR GB IT LI NL**

(56) Entgegenhaltungen:
**EP-A- 0 143 245**
**DE-B- 2 023 786**
**FR-A- 2 183 875**

**TENSIDE, Band 4, Nr. 1, 1967, Seiten 12-15, München, DE; N. EBERLE et al: "Herstellung und Einsatzmöglichkeiten von Mono- und Diglyceridgemischen natürlicher Fettsäuren als Emulgatoren für kosmetische Erzeugnisse"**

(73) Patentinhaber: **Th. Goldschmidt AG**
**Goldschmidtstrasse 100 Postfach 101461**
**W-4300 Essen 1(DE)**

(72) Erfinder: **Bade, Volkbert, Dr.**
**Graffweg 38 b**
**W-4300 Essen 14(DE)**

**EP 0 299 295 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Polyglycerinester der Wollfettsäure sowie Verfahren zur Herstellung dieser Verbindungen. Die Erfindung betrifft ferner die Verwendung dieser Verbindungen als Emulgatoren, insbesondere für kosmetische oder pharmazeutische Zwecke.

Zur Herstellung physiologisch einwandfreier und deshalb für kosmetische oder pharmazeutische Zwekke gut einsetzbarer Emulsionen vom Typ O/W oder W/O verwendet man seit vielen Jahrzehnten vollständige oder partielle Fettsäureester von mehrwertigen Alkoholen.

Die hierzu benötigten Fettsäuren wurden durch Spaltung natürlicher Fette und Öle gewonnen. Dabei werden im allgemeinen Fettsäuren mit 8 bis 18 Kohlenstoffatomen oder entsprechende Gemische von Fettsäuren verwendet. Die Fettsäuren können gesättigt oder ungesättigt und gegebenenfalls substituiert sein, wie z.B. die eine OH-Gruppe aufweisende Ricinolsäure. Besonders bevorzugt werden die Fettsäuren mit 10 bis 18 Kohlenstoffatomen oder deren bei der Spaltung natürlicher Fette erhaltenen Gemische zur Veresterung eingesetzt. Beispiele solcher Fettsäuregemische sind die Kokosfettsäuren, die durch Spaltung von Kokosfett erhalten werden. Die Fettsäuregemische können durch Hydrierung gehärtet sein.

Ein weiterer Rohstoff, aus dem veresterungsfähige Carbonsäuren gewonnen werden können und der in erheblicher Menge zur Verfügung steht, ist Wollfett, das auch als Wollwachs bezeichnet wird. Wollfett ist eine klar schmelzende, zähe, braungelbe bis schwarze, bockartig riechende Masse und wird aus roher Schafwolle gewonnen. Durch Esterspaltung erhält man die Wollfettsäuren (= Wollwachsfettsäuren). Es handelt sich dabei um ein sehr komplexes Gemisch gerad- und verzweigtkettiger Fettsäuren. Untersuchungen von Downing et al. in "Aust. J. Chem." 13 (1960), 80 ff. bewiesen das Vorliegen folgender Klassen von Carbonsäuren:

1. Normalreihe:
   - a) gesättigte unsubstituierte Carbonsäuren, etwa 10 Gew.-%
   - b) α-Hydroxycarbonsäuren, etwa 15 Gew.-%
   - c) ω-Hydroxycarbonsäuren, etwa 3,5 Gew.-%
2. Isoreihe:
   - a) $CH_3-CH(CH_3)(CH_2)_nCH_2COOH$, n = 7 bis 25, etwa 19 Gew.-%
   - b) α-Hydroxycarbonsäuren, etwa 12 Gew.-%
   - c) ω-Hydroxycarbonsäuren, etwa 0,5 Gew.-%
3. Anteisoreihe:
   - $CH_3CH_2CH(CH_3)(CH_2)_nCH_2COOH$, n = 7 bis 27, etwa 27 Gew.-%

Als Polyalkohol verwendete man zunächst das bei der Fettspaltung anfallende Glycerin, so daß die partiellen Fettsäureester des Glycerins, gegebenenfalls in Mischung mit Fettsäuretriglyceriden, zu den ältesten Emulgatoren zählen, die in die Kosmetik Eingang gefunden haben. Die Palette brauchbarer und physiologisch gut verträglicher Polyalkohole wurde durch die industrielle Herstellung des Hexaalkohols Sorbit durch Hydrierung von Glucose oder durch Hydrogenolyse von Rohrzucker oder Stärke wesentlich erweitert. Insbesondere die Laurinsäure-, Palmitin-/Stearinsäure- und Ölsäuremonoester des Sorbits werden in großem Umfang zur Herstellung kosmetischer und pharmazeutischer Emulsionen verwendet.

Als weitere Polyolkomponente werden die Polyglycerine verwendet. Die Polyglycerine werden üblicherweise durch Veretherung (Kondensation) von Glycerin bei erhöhten Temperaturen und in Gegenwart von Katalysatoren hergestellt. Die entstehenden Kondensationsprodukte sind vorzugsweise linear und entsprechen folgender Formel:

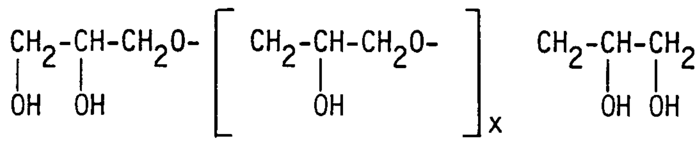

Im Falle des Diglycerins ist der Index x = 0. Bei der Bildung höherer Polyglycerine kann der Index x insbesondere einen Wert von 1 bis 10 annehmen.

Die Polyglycerine können mit üblichen Fettsäuren zu den entsprechenden Estern umgesetzt werden, wobei die Hydrophobie bzw. die Hydrophilie der Produkte durch das Ausmaß der Veresterung der zur Verfügung stehenden Hydroxylgruppen bestimmt wird. Synthese, Charakterisierung und Anwendung von Polyglycerin und Polyglycerinfettsäureestern sind in der Zeitschrift "Die Nahrung" 28 (1984), Seiten 815 bis 835, ausführlich beschrieben. Es wird dort angegeben, daß die Polyglycerinfettsäureester vielseitige Emulgatoren sind, die in der Lebensmittelindustrie, Medizin und Diätetik angewendet werden können. Sie werden insbesondere auf dem Lebensmittelsektor zur Herstellung von streichfähigen, eßbaren Fetten sowie zur Herstellung von Bratfetten eingesetzt. Die Polyglycerinfettsäureester verhindern dabei u.a. das Spritzen des erhitzten Fettes.

Die Polyglycerinfettsäureester werden ferner als Emulgatoren für Aufschlagmittel zur Herstellung von Biskuitbackwaren verwendet. Polyglycerinfettsäureester haben auch Eingang in die kosmetische und pharmazeutische Industrie gefunden. Derartige Anwendungen können der Zeitschrift "J. Soc. Cos. Chem." 15 (1964), Seiten 473 bis 483 entnommen werden. Als besonders brauchbar haben sich Decaglycerine erwiesen, bei denen 4 bis 10 Hydroxylgruppen mit Ölsäure oder Stearinsäure verestert sind. Diese Produkte werden auch zum Verdicken von mineralischen und pflanzlichen Ölen verwendet.

Emulgatoren auf der Basis von Estern, die als Säurekomponente Wollfettsäuren und als Polyolkomponente Glycerin enthalten, sind aus der DE-OS 20 23 786 bekannt. Sie dienen dort als Emulgatoren zur Herstellung von W/O-Emulsionen. Sie Können als partielle Ester oder als vollständige. Ester, gegebenenfalls zusammen mit den Wollfettalkoholen (= Wollwachsalkoholen) verwendet werden. Partielle Glyceride mit einem Veresterungsgrad von 40 bis 60 % werden als bevorzugt genannt. Die gebildeten W/O-Emulsionen sind stabil und beständig und können zur Herstellung kosmetischer Präparate verwendet werden.

Emulgatoren auf der Basis von mit Wollfettsäuren veresterten Polyglyceriden sind noch nicht beschrieben worden. Man erwartete von diesen Verbindungen offenbar keine besonderen und über das übliche Maß hinausgehenden Eigenschaften. Überraschenderweise wurde aber nun gefunden, daß bestimmte Polyglycerin-Wollfettsäure-Ester besonders stabile und lagerfähige W/O-Emulsionen herzustellen gestatten und in der Lage sind, auch solche Inhaltsstoffe in Emulsionen einzubringen, die als besonders schwer zu emulgieren bekannt sind. Beispiele solcher oft schwer zu emulgierender Substanzen sind insbesondere Parfümöle, wie $\alpha$- und $\gamma$-Terpineol.

Ein Gegenstand der vorliegenden Erfindung sind deshalb neue Polyglycerin-Wollfettsäure-Ester der allgemeinen Formel

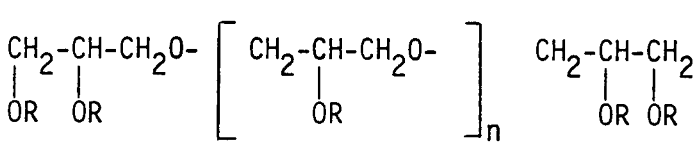

I

wobei R jeweils Wasserstoff- oder von der Wollfettsäure abgeleitete Alkoylreste bedeutet und die Bedingung erfüllt sein muß, daß mindestens ein Rest R die Bedeutung eines solchen Alkoylrestes hat, und n = 0 bis 12 ist.

Besonders bevorzugt sind Polyglycerin-Wollfettsäure-Ester, bei denen der Index n einen Wert von 1 bis 8, insbesondere 4 bis 8, hat.

Die besten Emulgiereigenschaften werden erhalten, wenn mindestens 50 % der Reste R von der Wollfettsäure abgeleitete Alkoylreste sind.

Die erfindungsgemäßen Polyglycerin-Wollfettsäure-Ester sind wachsartige bis feste, gelbe bis braune Verbindungen. Sie können durch bei der Herstellung von Fettsäureestern übliche oxidative Behandlung und/oder adsorptiv, z.B. durch Aktivkohle oder Bleicherde farblich aufgehellt werden. Die Schmelzpunkte der Ester liegen im allgemeinen zwischen 35 und 60 ° C.

Die erfindungsgemäßen Polyglycerin-Wollfettsäure-Ester weisen die gewünschte hohe Emulgierfähigkeit auch gegenüber schwer zu emulgierenden Substanzen auf und ermöglichen die Herstellung stabiler und lagerfähiger W/O-Emulsionen. Dieses Emulgierverhalten war aus den Eigenschaften der Wollfettsäure bzw. des Polyglycerins oder der bekannten Wollfettsäureester des Glycerins nicht herzuleiten.

Ein weiterer Vorteil der erfindungsgemäßen Verbindungen besteht in der Möglichkeit, Produkte unterschiedlichen Molekulargewichtes, aber gleichen oder ähnlichen HLB-Wertes herzustellen. So hat beispielsweise ein erfindungsgemäßer Ester mit einem Index n = 2, bei dem 50 % der Hydroxylgruppen mit Wollfettsäure verestert sind, einen HLB-Wert von etwa 4,4. Ein erfindungsgemäßer Ester mit einem Index n = 6 und gleichem Veresterungsgrad weist einen HLB-Wert von etwa 4,9 auf. Durch den unterschiedlichen Kondensationsgrad beider Produkte und damit durch deren unterschiedliches Molekulargewicht (1385 : 2395) und die unterschiedliche Anzahl der Estergruppen im Molekül läßt sich der Emulgator in seiner Emulgierwirksamkeit dem zu emulgierenden Substrat optimal anpassen.

Die erfindungsgemäßen Polyglycerin-Wollfettsäure-Ester der oben genannten Formel können entsprechend einem weiteren Gegenstand der Erfindung dadurch hergestellt werden, daß man Polyglycerin der allgemeinen Formel

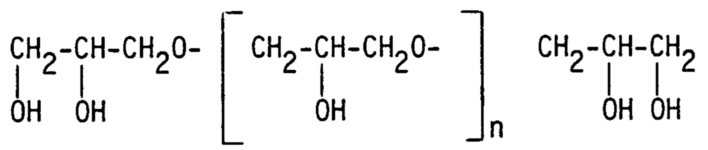

II

mit mindestens äquimolaren Mengen Wollfettsäure, vorzugsweise in Gegenwart von Veresterungskatalysatoren, in an sich bekannter Weise, bei Temperaturen von 140 bis 280 °C verestert.

Als Veresterungskatalysatoren können die aus dem Stand der Technik bekannten Katalysatoren, wie z.B. Alkalihydroxide, Alkalicarbonate, Alkalisalze von Fettsäuren, Verbindungen des Titans oder des Zinns, verwendet werden.

Das bei der Veresterung freiwerdende Wasser wird zweckmäßig in Verlauf der Reaktion abdestilliert, insbesondere azeotrop entfernt. Die Veresterungsreaktion läuft bei Temperaturen von 140 bis 280 °C ab. Sie verläuft in an sich bekannter Weise.

Es ist jedoch auch möglich, Wollfett direkt in nativer oder gereinigter Form mit Polyglycerin umzuestern. Ein weiterer Gegenstand der vorliegenden Erfindung besteht deshalb in einem Verfahren mit dem Kennzeichen, daß man Wollfett mit Polyglycerin der Formel II, vorzugsweise in Gegenwart von Umesterungskatalysatoren bei Temperaturen von 100 bis 300 °C in an sich bekannter Weise umestert und gegebenenfalls die freigesetzten Wollfettalkohole kontinuierlich oder nach der Umesterung in an sich bekannter Weise abtrennt. Es hat sich jedoch gezeigt, daß die recht aufwendige Entfernung der Wollfettalkohole bei Verwendung der erfindungsgemäßen Ester in der Kosmetik nicht immer erforderlich ist, da die Wollfettalkohole selbst gute emulgierende und hautpflegende Eigenschaften haben. Dies führt außerdem zu einer weiteren Verbilligung des Produktes.

Als Umesterungskatalysatoren können die weiter oben bereits genannten Katalysatoren des Standes der Technik verwendet werden, wobei die alkalisch reagierenden Katalysatoren bevorzugt sind.

Aufgrund der hervorragenden Emulgiereigenschaften und der physiologischen Unbedenklichkeit der erfindungsgemäßen Verbindungen besteht ein weiterer Gegenstand der Erfindung in ihrer Verwendung, gegebenenfalls im Gemisch mit Wollfettalkoholen, als Emulgatoren zur Herstellung von W/O-Emulsionen für kosmetische und pharmazeutische Zwecke. Die erfindungsgemäßen Emulgatoren lassen sich leicht verarbeiten und sind in geringer Menge wirksam. Sie weisen ihr gutes Emulgiervermögen auch gegenüber Verbindungen auf, deren schlechte Emulgierbarkeit bekannt ist. Hierzu gehören z.B. einige Parfümöle und pharmazeutisch wirksame Verbindungen. Die Zubereitungen lassen sich gut auf der Haut verteilen und ziehen schnell in die Haut ein. Die Verbindungen haben bereits selbst hautpflegende Eigenschaften, sind physiologisch unbedenklich und werden auch von empfindlicher Haut reizlos vertragen.

In den folgenden Beispielen werden die Herstellung erfindungsgemäßer Verbindungen, ihre Eigenschaften und ihre Verwendbarkeit näher gezeigt.

Zur Herstellung der erfindungsgemäßen Verbindungen der Formel I werden Polyglycerine der Formel II mit folgenden Kennzahlen verwendet:

| Polyglycerin | Wert n | OH-Zahl |
|---|---|---|
| Triglycerin | 1 | 1160 - 1197 |
| Hexaglycerin | 4 | 960 - 1000 |
| Decaglycerin | 8 | 885 - 890 |

Es wird Wollfettsäure folgender Kennzeichnung verwendet:

| | |
|---|---|
| Verseifungszahl | 140 - 185 |
| Säurezahl | 120 - 165 |
| Hydroxylzahl | 50 - 90 |
| Tropfpunkt *) | 45 - 60 °C |

*) nach Ubbelohde

Das verwendete Wollfett hat folgende Kennzahlen:

| | |
|---|---|
| Verseifungszahl | 88 - 102 |
| Säurezahl | max. 1,0 |
| Hydroxylzahl | 20 - 40 |
| Jodzahl | 18 - 32 |
| Unverseifbares | 45 - 55 |
| Tropfpunkt *) | 36 - 42 °C |

*) nach Ubbelohde

Herstellung erfindungsgemäßer Verbindungen der Formel I

1. Umsetzung von Polyglycerinen mit Wollfettsäuren

Herstellung von Triglycerin-tri-Wollfettsäure-Ester:
n = 1; 40 % der Reste R = H-, 60 % der Reste R = von der Wollfettsäure abgeleitete Alkoylreste

240 g Triglycerin (1 Mol) werden mit 1290 g Wollfettsäure (3 Mol) in einen Dreihalskolben mit Rührer, Einleitungsrohr, Kühlerbrücke und Vorlage eingewogen. Das Reaktionsgemisch wird unter $N_2$-Einleitung auf die Reaktionstemperatur von 250 bis 255 °C innerhalb einer Stunde aufgeheizt und 6 Stunden unter Abdestillieren der flüchtigen Reaktionsprodukte umgesetzt. Dabei sinkt die Säurezahl auf 3,0. Es werden 86 g Destillat aufgefangen.

Das Reaktionsprodukt hat eine braune bis dunkelbraune Farbe, wachsartige Konsistenz und folgende Kennzahlen:

| | |
|---|---|
| Säurezahl | 3,0 |
| Verseifungszahl | 120 |
| Hydroxylzahl | 84 |
| Tropfpunkt | 53 °C |

Herstellung von Hexaglycerin-hexa-Wollfettsäure-Ester:
n = 4; 25 % der Reste R = H-, 75% der Reste R = von der Wollfettsäure abgeleitete Alkoylreste

78 g Hexaglycerin (0,167 Mol) werden mit 480 g Wollfettsäure (1 Mol) wie im vorstehenden Beispiel verestert. Die Reaktionstemperatur beträgt 250 bis 252 °C. Am Ende der Reaktion beträgt die Säurezahl des Reaktionsproduktes 1,0. Es werden 34 g Destillat aufgefangen.

Das braune, wachsartige Reaktionsprodukt hat folgende Kennzahlen:

| | |
|---|---|
| Säurezahl | 1,0 |
| Verseifungszahl | 132 |
| Hydroxylzahl | 48 |
| Tropfpunkt | 51 °C |

Herstellung von Decaglycerin-octa-Wollfettsäure-Ester:
n = 8; 25 % der Reste R = H-, 75 % der Reste R = von der Wollfettsäure abgeleitete Alkoylreste

67 g Decaglycerin (0,087 Mol) werden mit 333 g Wollfettsäure (0,694 Mol) wie im vorstehenden Beispiel verestert. Am Ende der Reaktion beträgt die Säurezahl 1,0. Es werden 24 g Destillat aufgefangen.

Das braune, wachsartige Reaktionsprodukt hat folgende Kennzahlen:

| | |
|---|---|
| Säurezahl | 1,0 |
| Verseifungszahl | 120 |
| Hydroxylzahl | 67 |
| Tropfpunkt | 52 °C |

In der vorbeschriebenen Weise werden weitere erfindungsgemäße Verbindungen hergestellt, deren Kennzahlen in der folgenden Tabelle zusammengestellt sind:

| n = | % Reste R = Alkoyl | Säurezahl | Verseifungszahl | Hydroxylzahl | Tropfpunkt |
|---|---|---|---|---|---|
| 1 | 20 | 1,8 | 96 | 350 | 57 |
| 1 | 40 | 2,0 | 116 | 162 | 54 |
| 1 | 60 | 3,0 | 120 | 84 | 51 |
| 1 | 80 | 4,0 | 126 | 42 | 51 |
| 4 | 12,5 | 0,9 | 75 | 448 | 45 |
| 4 | 37,5 | 1,0 | 113 | 171 | 42 |
| 4 | 62,5 | 1,2 | 128 | 76 | 39 |
| 4 | 75 | 1,0 | 132 | 62 | 39 |
| 4 | 87,5 | 0,9 | 134 | 42 | 48 |
| 4 | 100 | 1,0 | 142 | 36 | 48 |
| 8 | 8,3 | 1,1 | 64 | 542 | 55 |
| 8 | 25 | 1,5 | 89 | 244 | 55 |
| 8 | 33,3 | 1,0 | 107 | 134 | 53 |
| 8 | 41,6 | 1,6 | 108 | 143 | 53 |
| 8 | 50 | 2,6 | 132 | 104 | 50 |
| 8 | 66,6 | 2,6 | 121 | 60 | 51 |
| 8 | 83,3 | 1,0 | 123 | 58 | 51 |
| 8 | 100 | 0,9 | 127 | 50 | 51 |

2. Umsetzung von Polyglycerinen mit Wollfett

Herstellung von Decaglycerin-Wollfett-Ester (ohne Katalysator)

108,5 g Decaglycerin werden mit 685 g Wollfett in einen Dreihalskolben mit Rührer, Einleitungsrohr, Kühlerbrücke und Vorlage eingewogen. Das Reaktionsgemisch wird innerhalb einer Stunde unter $N_2$-Einleitung auf eine Reaktionstemperatur von 240 bis 250 °C aufgeheizt und ohne Katalysatorzusatz 20 Stunden bei dieser Temperatur gehalten. Es fällt kein Destillat an.

Das erhaltene braune, wachsartige Produkt hat folgende Kennzahlen:

| | |
|---|---|
| Säurezahl | 0,4 |
| Verseifungszahl | 51 |
| Hydroxylzahl | 68 |
| Tropfpunkt | 40 ° C |

Herstellung von Decaglycerin-Wollfett-Ester (mit Katalysator)

259 g Wollfett und 41 g Decaglycerin werden mit 1,5 g Na-Stearat als Katalysator in eine wie oben beschriebene Apparatur eingewogen. Das Reaktionsgemisch wird 1 Stunde unter $N_2$-Einleitung auf die Reaktionstemperatur von 240 bis 250 ° C aufgeheizt und bei dieser Temperatur 2,5 Stunden lang umgesetzt. Es werden 1,5 g Na-Methylat als weiterer Katalysator zugegeben. Die Reaktion wird weitere 2 Stunden fortgesetzt. Es fallen 6,2 g Destillat an.

Das Reaktionsprodukt ist dunkelbraun und wachsartig mit folgenden Kennzahlen:

| | |
|---|---|
| Säurezahl | 0,9 |
| Verseifungszahl | 50 |
| Hydroxylzahl | 49 |
| Tropfpunkt | 41 ° C |

Herstellung von Decaglycerin-Wollfett-Ester (mit Katalysator)

129,5 g Wollfett und 20,5 g Decaglycerin werden mit 1,5 g Na-Methylat versetzt und in der oben beschriebenen Apparatur umgesetzt. Die Umsetzung wird bei 290 bis 300 ° C und einer Reaktionszeit von 2 Stunden durchgeführt. Es werden 6,8 g Destillat erhalten.

Das braune, wachsartige Umsetzungsprodukt hat folgende Kennzahlen:

| | |
|---|---|
| Säurezahl | 1,4 |
| Verseifungszahl | 42 |
| Hydroxylzahl | 29 |
| Tropfpunkt | 40 ° C |

Die anwendungstechnischen Eigenschaften der erfindungsgemäßen Verbindungen werden nach folgenden Verfahren geprüft:

Emulgierzahl:

Die Emulgierzahl ist ein Maß für das Wasseraufnahmevermögen eines Emulgators und gibt an, wieviel ml Wasser in 100 g einer 5 %igen Lösung des Emulgators in Paraffinöl einemulgiert werden können.

Ausführung:

1,0 g des zu prüfenden Emulgators in 19,0 g Paraffinöl perliquidum (DAB 9) in der Wärme lösen. Davon dann 10 g in ein mechanisches Mischgerät füllen und auf 50 ° C temperieren. Anschließend bei 150 U/min 5 ml 50 ° C warmes Wasser unter ständigem Rühren zugeben.

Die Zugabe wird in Abständen von jeweils einer Minute so lange fortgesetzt, bis von der gebildeten Emulsion kein Wasser mehr aufgenommen wird.

Emulgierzahl = verbrauchte ml $H_2O$ x 10

Parfümzahl:

Die Parfümzahl ist ein Maß für die Parfümaufnahmefähigkeit einer Emulsion und gibt an, wieviel ml Parfüm in 100 g einer Emulsion (Creme/Lotion) einemulgiert werden können. Die Zusammensetzung der Creme ist weiter unten angegeben.

Ausführung:

100 g Emulsion werden bei Raumtemperatur in ein Handemulgiergerät (Stampfgerät) eingewogen. Danach gibt man in 0,5-ml-Schritten Parfümöl zu und stampft jeweils 30mal. Die Creme wird dann auf Öl- und Wasseraustritt beurteilt. Der Vorgang wird so lange wiederholt, bis die Creme kein Parfüm mehr aufnehmen kann, ohne Wasser oder Öl auszuscheiden.
Parfümzahl = verbrauchte ml Parfümöl

| Creme-Rezeptur zur Bestimmung der Parfümzahl und Emulsionsstabilität | |
|---|---|
| Fettphase: | in Gew.-Teilen |
| Paraffinöl | 25 |
| Vaseline | 2 |
| Tafelparaffin (Tropfpunkt 105 ° C) | 2 |
| Ceresin | 3 |
| Cetyl-Stearylalkohol | 1,5 |
| Emulgator | 2,0 |
| Wasserphase: | |
| Wasser | 60,8 |
| $Mg_2SO_4 \cdot 7 H_2O$ | 0,5 |
| Glycerin | 3,0 |
| Parfümöl | 0,2 |

Ausführung:

Die Fettphase wird bei 105 ° C vermischt und dann in ein Handemulgiergerät (Stampfgerät) eingewogen. Nach dem Abkühlen auf 75 ° C wird die Wasserphase zugegeben und die gebildete Creme homogenisiert. Nach dem Abkühlen auf 40 ° C und der Zugabe des Parfümöles wird unter Vakuum 50mal gestampft. Das Gerät wird belüftet, erneut evakuiert und wieder 50mal gestampft. Die Lagerstabilität der Creme wird nach 24 Stunden Lagerung bei +40, -10, -20 ° C und Raumtemperatur beurteilt. Dabei bedeuten
\+ keine Öl- und/oder Wasserabscheidung
\- Öl- und/oder Wasserabscheidung

| 1. Durch Veresterung erhaltene Produkte | | | | | | | |
|---|---|---|---|---|---|---|---|
| n = | % Reste R = Alkoylreste | Emulgierzahl | Parfümzahl | Lagerstabilität | | | |
| | | | | +40°C | -10°C | -20°C | RT |
| 1 | 20 | 525 | 6,75 | - | + | - | - |
| 1 | 40 | 725 | 6,0 | - | + | - | + |
| 1 | 60 | 800 | 7,0 | + | + | + | + |
| 1 | 80 | 625 | 6,0 | + | + | - | + |
| 4 | 12,5 | 275 | 5,25 | - | - | - | - |
| 4 | 37,5 | 575 | 6,0 | - | + | - | + |
| 4 | 62,5 | 725 | 7,0 | + | + | + | + |
| 4 | 75 | 750 | 8,5 | + | + | + | + |
| 4 | 87,5 | 700 | 8,25 | + | + | + | + |
| 4 | 100 | 500 | 8,0 | + | + | - | + |
| 8 | 8,3 | 450 | 5,0 | - | + | - | + |
| 8 | 25 | 625 | 5,5 | + | + | - | + |
| 8 | 33,3 | 700 | 6,0 | + | + | + | + |
| 8 | 41,6 | 925 | 8,5 | + | + | + | + |
| 8 | 50 | 850 | ca. 35 | + | + | + | + |
| 8 | 66,6 | 900 | ca. 20 | + | + | + | + |
| 8 | 83,3 | 700 | ca. 20 | + | + | + | + |
| 8 | 100 | 550 | ca. 15 | - | - | - | + |

| 2. Durch Umesterung erhaltene Produkte | | | | | | |
|---|---|---|---|---|---|---|
| Umesterung des Wollfettes mit | Emulgierzahl | Parfümzahl | Lagerstablität | | | |
| | | | +40°C | -10°C | -20°C | RT |
| Decaglycerin ohne Katalysator | 800 | 20 | + | + | + | + |
| Decaglycerin mit Katalysator Reaktionstemp. 250°C | 850 | 24 | + | + | + | + |
| Decaglycerin mit Katalysator Reaktionstemp. 290°C | 700 | 34 | + | + | + | + |
| 3. Vergleichswerte | | | | | | |
| Wollfett | 450 | 4,0 | - | - | - | - |
| Wollfettalkohol | 550 | 3,0 | - | + | + | + |
| Wollfett/Decaglycerin als Gemisch | inhomogen, Bodensatz, Messung nicht möglich | | | | | |

## Patentansprüche

**1.** Polyglycerin-Wollfettsäure-Ester der allgemeinen Formel

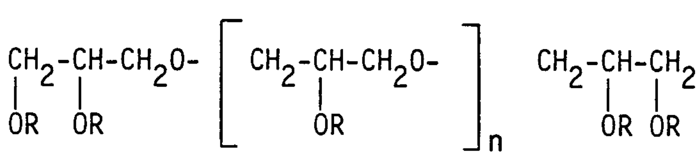

wobei R jeweils Wasserstoff- oder von der Wollfettsäure abgeleitete Alkoylreste bedeutet und die Bedingung erfüllt sein muß, daß mindestens ein Rest R die Bedeutung eines solchen Alkoylrestes hat, und n = 0 bis 12 ist.

**2.** Polyglycerin-Wollfettsäure-Ester nach Anspruch 1, dadurch gekennzeichnet, daß mindestens 50 % der Reste R von der Wollfettsäure abgeleitete Alkoylreste sind und n = 4 bis 8 ist.

**3.** Verfahren zur Herstellung der Verbindungen des Anspruchs 1 oder 2, dadurch gekennzeichnet, daß man Polyglycerin der allgemeinen Formel

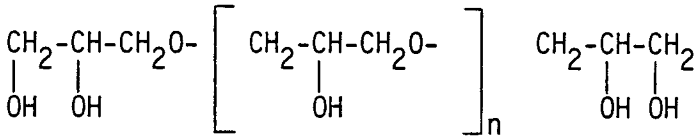

mit mindestens äquimolaren Mengen Wollfettsäure, vorzugsweise in Gegenwart von Veresterungskatalysatoren, in an sich bekannter Weise, bei Temperaturen von 140 bis 280 ° C verestert.

**4.** Verfahren zur Herstellung der Verbindungen des Anspruchs 1, dadurch gekennzeichnet, daß man Wollfett mit Polyglycerin der allgemeinen Formel

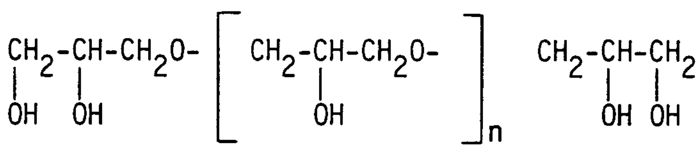

vorzugsweise in Gegenwart von Umesterungskatalysatoren bei Temperaturen von 100 bis 300 ° C in an sich bekannter Weise umestert und gegebenenfalls die freigesetzten Wollfettalkohole kontinuierlich oder nach der Umesterung in an sich bekannter Weise abtrennt.

**5.** Verwendung der Verbindungen des Anspruchs 1 oder 2 oder ihrer Gemische mit Wollfettalkoholen als Emulgatoren, insbesondere für kosmetische oder pharmazeutische Zwecke.

**Claims**

**1.** Polyglycerol-wool fatty acid asters of the general formula

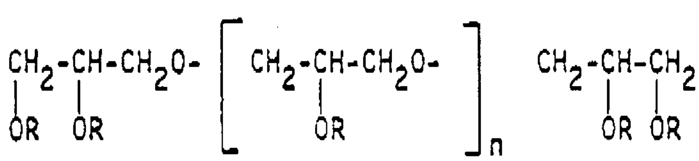

wherein R in each case denotes hydrogen radicals or alkoyl radicals derived from wool fatty acid, and the condition that at least one radical R denotes such an alkoyl radical must be met, and n is 0 to 12.

**2.** Polyglycerol-wool fatty acid esters according to Claim 1, characterised in that it least 50% of the radicals R are alkoyl radicals derived from wool fatty acid and n is 4 to 8.

**3.** Process for the preparation of the compounds of Claim 1 or 2, characterised in that polyglycerol of the general formula

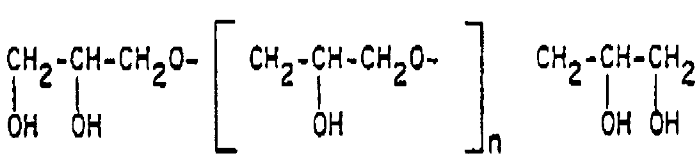

is esterified with at least equimolar amounts of wool fatty acid, preferably in the presence of esterification catalysts, in a manner which is known per se, at temperatures from 140 to 280 ° C.

**4.** Process for the preparation of the compounds of Claim 1, characterised in that wool fat is transesterified with polyglycerol of the general formula

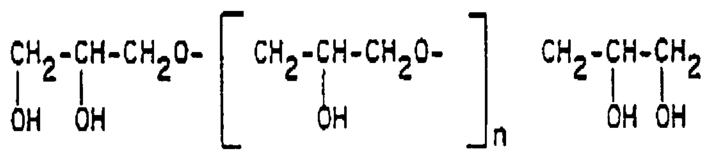

preferably in the presence of transesterification catalysts at temperatures from 100 to 300 ° C in a manner which is known per se, and if appropriate the wool fat alcohols liberated are removed, in a manner which is known per se, continuously or after the transesterification.

**5.** Use of the compounds of Claim 1 or 2 or their mixtures with wool fat alcohols as emulsifiers, in particular for cosmetics or pharmaceutical purposes.

**Revendications**

**1.** Esters de polyglycérol et d'acide de lanoline répondant à la formule générale

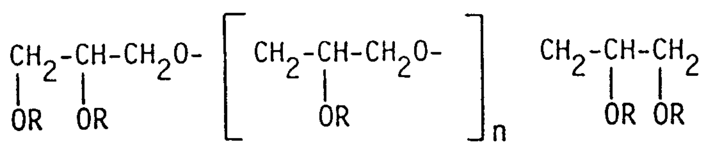

où R représente à chaque fois un atome d'hydrogène ou des restes alcoyle dérivés de l'acide de lanoline, la condition à remplir étant qu'au moins un reste R représente un tel reste alcoyle, et n = 0 à 12.

**2.** Esters de polyglycérol et d'acide de lanoline selon la revendication 1, caractérisés en ce qu'au moins 50 % des restes R sont des restes alcoyle dérivés de l'acide de lanoline et n = 4 à 8.

**3.** Procédé de préparation des composés selon la revendication 1 ou 2, caractérisé en ce qu'on estérifie un polyglycérol de formule générale

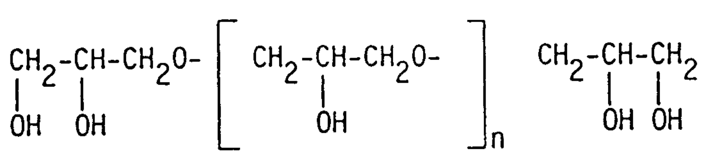

avec des quantités au moins équimolaires d'acide de lanoline, de préférence en présence de catalyseurs d'estérification, d'une manière connue en soi, à une température comprise entre 140 et 280 ° C.

**4.** Procédé de préparation des composés selon la revendication 1, caractérisé en ce qu'on transestérifie la lanoline avec un polyglycérol de formule générale

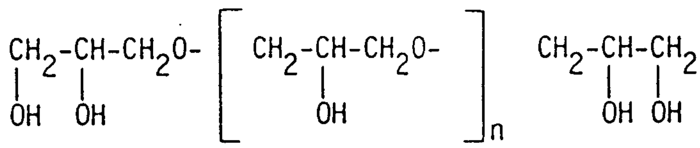

de préférence en présence de catalyseurs de transestérification, à une température comprise entre 100 et 300 ° C, d'une manière connue en soi, et on sépare éventuellement les alcools de lanoline libérés, en continu ou après la transestérification, d'une manière connue en soi.

**5.** Utilisation des composés selon la revendication 1 ou 2, ou de leurs mélanges, avec des alcools de lanoline comme émulsifiants, en particulier à des fins cosmétiques ou pharmaceutiques.